# EUROPÄISCHE PATENTSCHRIFT

(11) **EP 2 872 241 B1**
(45) Veröffentlichungstag und Bekanntmachung des Hinweises auf die Patenterteilung: **23.11.2016**
(21) Anmeldenummer: 13782649.1
(22) Anmeldetag: 22.08.2013
(51) Int. Cl.: C07C 29/78, C10G 29/20, C10G 3/00, C07C 41/09, C07C 41/42, F17D 1/16, F17D 1/17, C07C 29/151

(54) **VERFAHREN ZUR VERBESSERUNG DER TRANSPORTFÄHIGKEIT VON SCHWEREM ROHÖL**
METHOD FOR IMPROVING THE TRANSPORTABILITY OF HEAVY CRUDE OIL
PROCÉDÉ D'AMÉLIORATION DE LA CAPACITÉ DE TRANSPORT DE BRUT LOURD

(43) Veröffentlichungstag der Anmeldung: 20.05.2015
(73) Patentinhaber: Wagner, Ulrich, Dr., 06408 Biendorf (DE); Balthasar, Wolff, 40833 Ratingen (DE); Müller, Dierk, 61184 Karben (DE)
(72) Erfinder: WAGNER, Ulrich, 06406 Bernburg (DE); BALTHASAR, Wolff, 40833 Ratingen (DE); MÜLLER, Dierk, 61184 Karben (DE)
(74) Vertreter: Söylemezoglu, Mustafa Nazim
(86) Internationale Anmeldenummer: PCT/DE2013/100304
(87) Internationale Veröffentlichungsnummer: WO 2015/024539

(56) Entgegenhaltungen:
- DE-A1- 2 451 342
- NL-A- 7 500 545
- US-A- 3 870 063
- US-A- 4 076 761
- US-A1- 2005 197 412
- US-A1- 2009 014 336

## Beschreibung

Die Erfindung bezieht sich auf ein Verfahren zur Verbesserung der Transportfähigkeit von schwerem Rohöl.

Es ist allgemein bekannt, in einer Erdöllagerstätte gefördertes Erdöl als Rohöl zur weiteren Verarbeitung in einer Raffinerie über Rohrleitungen zu transportieren, wobei sich diese über Entfernungen von mehreren tausend Kilometern erstrecken können.

Lassen sich in einer Erdöllagerstätte nur noch schwere oder superschwere Rohöle gewinnen, also solche z.B. mit einer Viskosität von in etwa 40 000 mPa s bzw. 24° API (API = amerikanische Dichteeinheit für Rohöl), so ist ein längerer Transport derartiger Rohöle durch Rohrleitungen nicht mehr möglich bzw. unwirtschaftlich.

Es wurde daher bereits nach verschiedenen Möglichkeiten gesucht, die Transportfähigkeit von schwerem Rohöl, insbesondere durch Verringerung der Viskosität, zu verbessern. Aus der DE 36 09 641 A1 ist bekannt, zum Transport von zähfließendem Rohöl dieses in eine Öl-in-Wasser-Emulsion mit mindestens 10 bis 15 % Wasser unter Zusatz eines speziellen Emulgators auf Basis Oxethylat umzuwandeln.

Gemäß WO 2011/006024 A2 wird zur Verringerung der Viskosität vorgeschlagen, ein Polymer bestehend aus einem nicht-ionischen Monomeren und mindestens 25 Molprozent kationischer Monomere einzusetzen. Der Zusatz von Emulgatoren oder Polymeren als Verdünnungsmittel ist mit zusätzlichen Kosten verbunden und erfordert, dass diese vor der Raffination des Rohöls wieder entfernt werden müssen.

In der DE 2 039 329 A wird zur Transportverbesserung vorgeschlagen, Rohöl auf Temperaturen von 340 bis 650 °C zu erhitzen. Dies ist jedoch mit einem erheblichen Aufwand verbunden und bei Transportstrecken von mehreren tausend Kilometern wirtschaftlich nicht realisierbar.

In der US 7,861,737 B2 wird vorgeschlagen, zur Transportverbesserung von Schweröl diesem erst ein Lösungsmittel, wie z.B. Naphtha, zuzusetzen, um das Schwer- bzw. Rohöl zu verdünnen. Danach wird Dimethylether (DME) in flüssigem oder gasförmigem Zustand unter Druck, mindesten 4 bar, eingetragen. Der Zusatz von DME soll zu einer deutlichen Verringerung der Viskosität des Schwer- bzw. Rohöls führen, wodurch die Transportfähigkeit verbessert wird. Der Nachteil dieser Lösung ist, dass zwei Komponenten, Naphtha und DME, bereitgestellt, zur Erdöllagerstätte transportiert und dem Schwer- bzw. Rohöl zugesetzt werden müssen. Der Zusatz von DME als Komponente mit einem hohen Partialdruck ist mit zusätzlichem Aufwand beim Mischen und Verpumpen verbunden.

In der US 7,861,737 82 wird vorgeschlagen, zur Transportverbesserung von Schweröl diesem erst ein Lösungsmittel, wie z.B. Naphtha, zuzusetzen, um das Schwer- bzw. Rohöl zu verdünnen. Danach wird Dimethylether (DME) in flüssigem oder gasförmigem Zustand unter Druck, mindesten 4 bar, eingetragen. Der Zusatz von DME soll zu einer deutlichen Verringerung der Viskosität des Schwer- bzw. Rohöls führen, wodurch die Transportfähigkeit verbessert wird. Der Nachteil dieser Lösung ist, dass zwei Komponenten, Naphtha und DME, bereitgestellt, zur Erdöllagerstätte transportiert und dem Schwer- bzw. Rohöl zugesetzt werden müssen. Der Zusatz von DME als Komponente mit einem hohen Partialdruck ist mit zusätzlichem Aufwand beim Mischen und Verpumpen verbunden. Naphtha enthält überwiegend Cycloparaffine. Die Zusätze Naphtha und DME müssen während der nachfolgenden Raffination des Rohöls destillativ mit abgetrennt werden. DME ist unter Normalbedingungen ein hochentzündliches Gas. Der Umgang mit diesem hochexplosiven Stoff erfordert erhebliche sicherheitstechnische Aufwendungen. Aufgabe der Erfindung ist es, ein Verfahren zur Verbesserung der Transportfähigkeit von schwerem Rohöl mittels eines Zusatzes zu schaffen, der aus bei der Erdölgewinnung anfallenden Haupt- und Nebenprodukten herstellbar ist, keine besonderen sicherheitstechnischen Vorkehrungen erfordert, mit geringem Aufwand dem schweren Rohöl zugesetzt werden kann, während der nachfolgenden Raffination nicht abgetrennt werden muss und zu einer erhöhten Ausbeute an herkömmlichem Benzin führt.

Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 18.

Zur Verbesserung der Transportfähigkeit von schwerem Rohöl wird diesem als viskositätserniedrigendes Mittel ein wasserhaltiges Kohlenwasserstoffgemisch mit einer Kettenlänge von vorwiegend C4 bis C12, das keine sauerstoffhaltigen Verbindungen enthält, zugesetzt. Vorwiegend bedeutet hier, dass ca. 80 bis 85 % der Kohlenwasserstoffe eine Kettenlänge von C4 bis C12 aufweisen. Bei den restlichen 15 bis 20 % handelt es sich um Verbindungen mit den Kettenlängen C3 bzw. > C12.

Das spezielle Kohlenwasserstoffgemisch wird im Bereich einer Erdöllagerstätte aus anfallendem Erdgas und/oder Erdölbegleitgas sowie aus einer Teilmenge an schwerem Rohöl hergestellt. Dabei werden parallel aus Erdgas und/oder Erdölbegleitgas sowie aus der abgezweigten Teilmenge an schwerem Rohöl jeweils als Zwischenprodukte ein erstes Synthesegas und ein zweites Synthesegas hergestellt, diese zusammengeführt, in einem Mischer vermischt und anschließend komprimiert. Das Zwischenprodukt "Synthesegasgemisch" wird katalytisch in Methanol und dieser durch Kondensation in ein Methanol-Wasser-Gemisch (Rohmethanol) umgewandelt, das zu einem Destillat mit einem hohen Wasser- und Alkoholgehalt von über 90 % aufgearbeitet und katalytisch in ein Dimethylether/Methanol/Wasser-Gemisch umgewandelt wird. Durch Dehydratation wird das wasserhaltige Kohlenwasserstoffgemisch mit einer Kettenlänge von vorwiegend C4 bis C12 gebildet. Dieses wird entweder unbehandelt oder nach Entgasung und/oder Entwässerung dem schweren Rohöl zugesetzt, wodurch aus dem schweren Rohöl ein in seiner Qualität leichtes Rohöl erhalten wird. Dieses wird über Leitungen zu einer Raffinerie transportiert und während der nachfolgenden Raffination des leichten Rohöls zu herkömmlichem Benzin die produzierte Menge an Benzin um die im wasserhaltigen Kohlenwasserstoffgemisch enthaltenen Kohlenwasserstoffe erhöht, zumindest um den Anteil, der auf das Erd- und/oder Erdölbegleitgas zurückzuführen ist.

Gemäß einer weiteren Ausgestaltung wird das erste Synthesegas wie folgt aus Erd- und/oder Erdölbegleitgas hergestellt:
a1) das Begleitgas wird entschwefelt und mit Prozesskondensat und Dampf gesättigt;
a2) das gemäß Verfahrensschritt a1) erhaltene Prozessgas wird in ein Gasgemisch aus Methan, Kohlendioxid und Kohlenmonoxid vorgespalten;
a3) das vorgespaltene Gasgemisch wird unter erhöhter Temperatur und unter Druck in einem Steamreformer katalytisch in ein erstes Synthesegas umgewandelt, das abgekühlt und mittels eines Kompressors komprimiert wird.

Das zweite Synthesegas wird aus der Teilmenge an schwerem Rohöl wie folgt hergestellt:
b1) aufbereitetes Rohöl wird erhitzt und mit vorgewärmtem Sauerstoff, der in einer Luftzerlegungsanlage gewonnen wird, sowie Prozessdampf vermischt,
b2) dieses Gemisch wird in einem POX-Reaktor mittels nichtkatalytischer partieller Oxidation in CO und Wasserstoff gespalten,
b3) nachfolgendes Quenchen mit Wasser und Wärmeabfuhr über eine Abhitzestrecke und
b4) abschließendes Abtrennen von Schwefelwasserstoffverbindungen aus dem Prozessgas.

Die Umwandlung des Synthesegases, das sich aus dem ersten und zweiten Synthesegas zusammensetzt, in Methanol erfolgt in einem dualen System, bestehend aus einem wassergekühlten und einem gasgekühlten Reaktor bei Temperaturen von 220 bis 260 °C.

Die beiden Synthesegase unterscheiden sich in ihrer Zusammensetzung.

Das Mischungsverhältnis zwischen dem ersten und zweiten Synthesegas sollte auf das in der Methanolsynthese erforderliche Verhältnis von Wasserstoff zu Kohlenmonoxid abgestimmt sein, wobei die aus dem Begleitgas stammende Menge an Synthesegas den größeren Anteil bildet.

Die Zusammensetzung des ersten Synthesegases kann im Bereich der Stöchiometriezahl von 2,8 bis 3,3 schwanken und die Zusammensetzung des zweiten Synthesegases im Bereich der Stöchiometriezahl von 0,8 bis 1,1.

Während der Methanolsynthese wird eine Teilmenge an Synthesegas ausgekreist, im Kreislauf gefahren und dabei auf den erforderlichen Betriebsdruck komprimiert.

Das kondensierte Rohmethanol wird entgast und nachfolgend von niedrig siedenden und höher siedenden Verbindungen gereinigt, wobei die destillative Reinigung bei Temperaturen von 70 bis 140 °C in zwei Kolonnen vorgenommen wird und sich im Methanol ein Restwassergehalt von mindestens 4 % einstellt.

Das im Festbettreaktor anfallende Dimethylether/Methanol/Wasser-Gemisch wird zur Temperatureinstellung mit Recyclegas versetzt.

Das zur Viskositätserniedrigung gebildete Kohlenwasserstoffgemisch wird unmittelbar, im Bereich der Erdöllagerstätte, entweder dem bereits geförderten und/oder dem noch unterirdisch gelagerten schweren Rohöl über das Bohrloch zugeführt oder über ein Spülrohr in das Bohrloch eingebracht.

Das gebildete Kohlenwasserstoffgemisch sollte vor dem Inkontaktbringen mit dem schweren Rohöl entwässert und entgast werden.

Aufbereitetes Kohlenwasserstoffgemisch, das entwässert und entgast ist, kann auch vor oder nach der zentralen Ölaufarbeitung dem schweren Rohöl zugesetzt werden.

Im Gegensatz dazu sollte der Zusatz von nicht aufbereitetem Kohlenwasserstoffgemisch bereits vor der zentralen Ölaufarbeitung erfolgen.

Vorzugsweise wird nach der Abtrennung von Wasser und Ölbegleitgas aus dem schweren Rohöl diesem eine weitere Menge an aufbereitetem Kohlenwasserstoffgemisch zugesetzt, die in Abhängigkeit von der Viskosität des Schweröls so dosiert wird, dass ein leichtes Rohöl gebildet wird.

Vorteilhaft ist es, aufbereitetes Kohlenwasserstoffgemisch auf der Saugseite der zum Transport des Rohöls eingesetzten Pumpe zuzuführen.

Gemäß einer weiteren Ausgestaltung des Verfahrens ist es wirtschaftlich von Vorteil, wenn die Viskosität des geförderten schweren Rohöls gemessen und in Abhängigkeit vom aktuellen Messergebnis die Menge an Kohlenwasserstoffgemisch dosiert zugesetzt wird, um leichtes Rohöl zu erhalten.

Das aufbereitete Kohlenwasserstoffgemisch und schweres Rohöl werden z.B. in einer separaten Mischeinrichtung unmittelbar vor dem Weitertransport vermischt.

Die Erfindung wird nachstehend an einem Beispiel näher erläutert.

In der zugehörigen Zeichnung ist die Verfahrensweise in einem Fließschema dargestellt. In einer Erdöllagerstätte werden 451,1 t/h an schwerem Rohöl (API 23°) gefördert, das folgende Zusammensetzung aufweist:
- Kohlenwasserstoffe 317,5 t
- Wasser 135,4 t, darin enthalten 15,1 t gasförmige Bestandteile und 13,5 t Schwefelverbindungen.

In einer zentralen Ölaufarbeitung 1 wird das aus unterschiedlichen Bohrlöchern 2 stammende Rohöl zusammengeführt, gemischt und nachfolgend einer Trenneinrichtung zugeführt, in der die wässrige Phase und gasförmige Bestandteile abgetrennt werden. Die Trenneinrichtung ist Bestandteil der zentralen Ölaufarbeitung 1. In dem Fließschema sind symbolhaft drei Bohrlöcher 2 dargestellt.

Im Zusammenhang mit der Erdölförderung fällt Erdgas/Erdölbegleitgas mit folgender Zusammensetzung an:
- Stickstoff 1,5 %
- Methan 92 %
- Ethan 3,5 %
- Propan 1,5 %
- höhere Kohlenwasserstoffe 1,5 %
- Schwefel 50 ppm.

Das Erdgas/Ölbegleitgas (70851 Nm³/h) sowie aufbereitetes Rohöl (47,6 t/h) werden in einer auf dem Gelände der Erdöllagerstätte errichteten Chemieanlage wie folgt in ein Kohlenwasserstoffgemisch umgewandelt.

Das Erdgas/Ölbegleitgas 3 wird bei einem Druck von 28 bar zunächst in einer Entschwefelungseinheit 4 bei einer Temperatur von 380 °C über einem Zinkoxidbett entschwefelt. Nach weiterer Aufheizung auf 560 °C wird das angefallene Prozessgas mit Dampf in einem Steamreformer 5, einem außenbeheizten Röhrenreaktor mit Nickel-Katalysator, bei einem Druck von 19,4 bar und 870 °C in ein Gemisch aus Wasserstoff, CO und CO₂ umgewandelt. Über verschiedene Stufen eines Abhitzesystem 6, die zur Dampferzeugung im Abhitzekessel, Kesselspeisewasservorwärmung, Vorwärmung des Erdgases/Ölbegleitgases sowie zum Heizen der Methanoldestillation genutzt werden, wird das nunmehr mit einem Druck von 18 bar anliegende und gekühlte erste Synthesegas (281900 Nm³) in einer ersten Stufe 8 eines Synthesegaskompressors auf 57,5 bar komprimiert.

Das erste Synthesegas hat folgende Zusammensetzung (Angaben in mol%):
CO₂ = 7,45; CO = 15,05; H₂ = 73,28; N₂ = 0,06; CH₄ = 4,15; H₂O = 0,01; (Stöchiometriezahl SZ = 2,926).

Parallel zur Umwandlung des Erdgas/Ölbegleitgases wird aus einer abgezweigten Teilmenge 7 (ca. 10 %) an Rohöl ein zweites Synthesegas, wie folgt hergestellt.

47,6 t/h Rohöl (enthält 33,3 t/h Kohlenwasserstoffe und 14,3 t/h Wasser) werden in einer Heizvorrichtung 10 auf 300 °C erhitzt und mit auf 230 °C vorgewärmten Sauerstoff, der in einer Luftzerlegungsanlage 25 gewonnen und über eine Leitung 26 zugeführt wird, sowie Prozessdampf vermischt. Dieses Gemisch wird in einem POX-Reaktor 11 mittels nichtkatalytischer partieller Oxidation bei 60 bar und 1400 °C hauptsächlich in CO und Wasserstoff gespalten. Nach Quenchen mit Wasser und Wärmeabfuhr über eine Abhitzestrecke 12 wird das auf 40 °C abgekühlte Gas in einem Wäscher 13 gewaschen, um Schwefelwasserstoffverbindungen abzutrennen. Diese werden in einer nicht näher gezeigten separaten Claus-Anlage in festen Schwefel umgewandelt.

Aus der eingesetzten Menge an Rohöl werden 108823 Nm³ an Synthesegas erhalten. Dieses zweite Synthesegas hat folgende Zusammensetzung (Angaben in mol%): CO₂ = 4,6; CO = 43,81; H₂ = 51,1; N₂ = 0,13, CH₄= 0,19; H₂O = 0,16 (Stöchiometriezahl SZ = 0,96).

Es unterscheidet sich in seiner Zusammensetzung von dem ersten Synthesegas.

Die erzeugte Menge an zweitem Synthesegas (z.B. 72 %) ist größer als die erzeugte Menge an erstem Synthesegas (z.B. 28 %).

In einem Mischer 14 werden das erste Synthesegas und das zweite Synthesegas miteinander vermischt und in einer zweiten Stufe 15 des Synthesegaskompressors auf 80 bar komprimiert.

Die jeweiligen Mengen der beiden zu vermischenden Synthesegase sind so aufeinander abgestimmt, dass in der nachfolgenden Methanolsynthese genau das richtige Verhältnis Wasserstoff zu Kohlenmonoxid vorliegt. In der Regel sollte das Verhältnis Wasserstoff zu Kohlenmonoxid in etwa 2:1 betragen. Unter diesen Bedingungen wird der Aufwand für die Methanolsynthese erheblich verringert, da die Synthese mit einem kleinen Recycleverhältnis von 2,0 betrieben werden kann.

Nachfolgend wird in einem dualen System, bestehend aus einem wassergekühlten und einem gasgekühlten Reaktor 18, das Synthesegasgemisch katalytisch im Temperaturbereich von 220 bis 260 °C in Methanol umgewandelt und durch Kondensation ein Rohmethanol 19 mit folgender Zusammensetzung erhalten:
- Methanol 84,7 Gew.-%,
- Kohlendioxid 3,6 Gew.-%,
- Wasser 10,0 Gew.-%,
- Methan 1,5 Gew.-%,
- höhere Kohlenwasserstoffe 0,1 Gew.-% und
- höhere Alkohole 0,1 %.

Während der Methanolsynthese wird über die Leitung 16 Recyclegas im Kreislauf gefahren und mittels eines Reyclegaskompressors 17 komprimiert.

Das nach der Methanolsynthese in mehreren Stufen kondensierte Rohmethanol 19 wird in einer nachgeschalteten Destillationsanlage 20 zunächst entgast und nachfolgend von niedrig siedenden sowie abschließend höher siedenden Produkten gereinigt. Gegenüber der klassischen dreistufigen Destillation zur Herstellung von marktfähigem Methanol wird die Destillation im Temperaturbereich von 70 bis 140 °C in nur zwei Kolonnen durchgeführt. Im erzeugten Methanol stellt sich ein Restwassergehalt von 4 % ein.

Insgesamt fallen nach der Destillation 167,6 t/h Rohmethanol an, die 6,7 t Wasser enthalten.

Das auf 4 % Wassergehalt destillierte Methanol wird nachfolgend in einem Festbettreaktor 21 (DME-Reaktor) katalytisch in ein DME (Dimethylether)/Methanol/Wasser-Gemisch umgewandelt.

Das Reaktionsprodukt aus dem DME-Reaktor wird mit Recyclegas, das über eine Leitung 22 aus der nachgeschalteten Entgasungseinheit entnommen wird, zur Temperatureinstellung versetzt und in weiteren adiabatisch arbeitenden Reaktoren 23 im Temperaturbereich von 320 bis 420 °C in ein sauerstofffreies Kohlenwasserstoff/Wasser-Gemisch umgewandelt. Aus den eingesetzten 167,6 t/h Methanol entstehen 70,6 t Kohlenwasserstoffe und 97 t Wasser. Dieses wasserhaltige Kohlenwasserstoffgemisch mit einer Kettenlänge, vorwiegend im Bereich C4 bis C12, enthält nach der Destillation keine sauerstoffhaltigen Verbindungen (DME bzw. Alkohole).

Es wird abschließend in einer Entgasungseinheit 24 entgast und dem unaufbereiteten schweren Rohöl zur Transportverbesserung zugesetzt.

Gemäß diesem Beispiel werden dem unaufbereiteten schweren Rohöl (403,4 t/h) kontinuierlich 167,6 t wässriges Kohlenwasserstoffgemisch pro Stunde zugemischt. Im Fließschema ist die Stelle des Zumischens mit dem Bezugszeichen 27 gekennzeichnet.

Die Zumischung des wässrigen Kohlenwasserstoffgemisches erfolgt vor dem Trennprozess Rohöl/Erdölbegleitgas, der innerhalb der zentralen Ölaufbereitung 1 stattfindet. Nachfolgend werden in der zentralen Ölaufbereitung 1 aus dem verdünnten Rohölgemisch die wässrige Phase und noch vorhandene gasförmige Bestandteile, wie Stickstoff, Kohlendioxid, Methan und Ethan, abgetrennt. Es werden 338 t/h aufbereitetes Rohöl mit einem API 36° erhalten. Dieses kann nunmehr in herkömmlichen Transportrohrleitungen 28 mit Pumpstationen über tausende von Kilometern ohne Probleme transportiert werden. Dieses modifizierte Rohöl besitzt eine Qualität wie leichtes Rohöl.

Der Vorteil bei der weiteren Verarbeitung bzw. Raffination des leichten Rohöls zu Benzin ist, dass die zur Verbesserung der Transportfähigkeit zugesetzten speziellen Kohlenwasserstoffe absolut keinen Nachteil auf das Raffinationsverfahren haben und als wirksamer Bestandteil des gewonnenen Benzins erhalten bleiben.

Neben der Transportverbesserung kann das gesamte schwere Rohöl zusätzlich dem aus dem Erdölbegleitgas gewonnenen Anteil an Kohlenwasserstoffen in herkömmliches Benzin umgewandelt werden.

Der erfindungsgemäße Zusatz muss nicht wieder entfernt werden und hat keine nachteiligen Einflüsse auf das Raffinationsverfahren und das Endprodukt (Benzin).

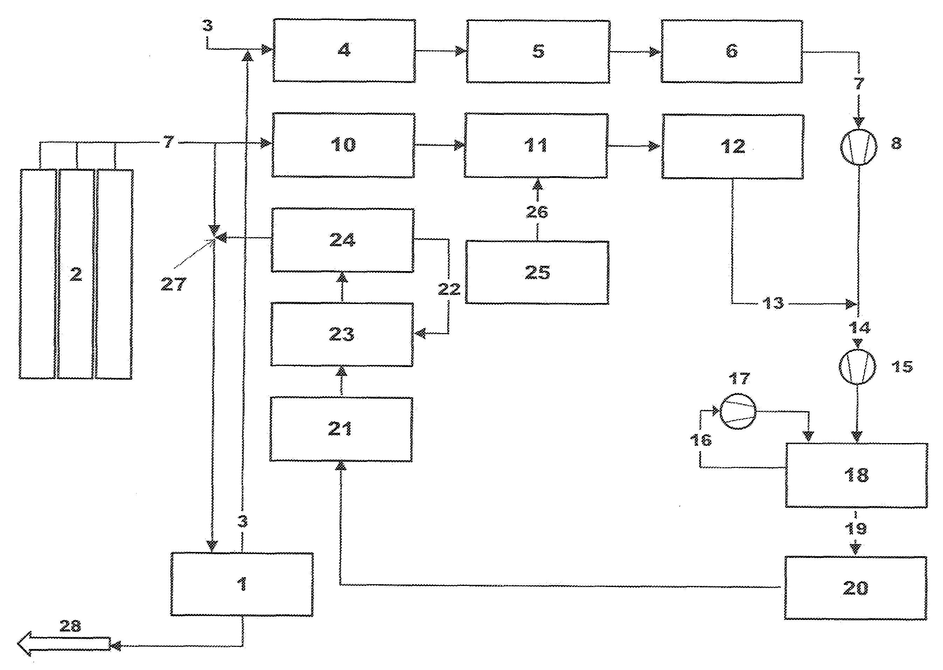

## Patentansprüche

1. Verfahren zur Verbesserung der Transportfähigkeit von schwerem Rohöl, wobei dem schweren Rohöl ein viskositätserniedrigendes Mittel zugesetzt wird, **dadurch gekennzeichnet, dass** das Mittel ein wasserhaltiges Kohlenwasserstoffgemisch mit einer Kettenlänge von vorwiegend C4 bis C12 ist, das keine sauerstoffhaltigen Verbindungen enthält, und im Bereich einer Erdöllagerstätte aus anfallendem Erdgas und/oder Erdölbegleitgas sowie aus einer Teilmenge an schwerem Rohöl hergestellt wird, wobei parallel aus Erdgas und/oder Erdölbegleitgas sowie aus der abgezweigten Teilmenge an schwerem Rohöl jeweils als Zwischenprodukte ein erstes Synthesegas und ein zweites Synthesegas hergestellt, zusammengeführt, in einem Mischer vermischt und anschließend komprimiert werden, und nachfolgend das Synthesegas katalytisch in Methanol und dieses durch Kondensation in ein Methanol-Wasser-Gemisch (Rohmethanol) umgewandelt wird, das zu einem Destillat mit einem hohen Wasser- und Alkoholgehalt von über 90 % aufgearbeitet und katalytisch in ein Dimethylether/Methanol/Wasser-Gemisch umgewandelt wird, und durch Dehydratation das wasserhaltige Kohlenwasserstoffgemisch mit einer Kettenlänge von C4 bis C12 gebildet wird, das entweder unbehandelt oder nach Entgasung und/oder Entwässerung dem schweren Rohöl zugesetzt wird, wodurch aus dem schweren Rohöl ein in seiner Qualität leichtes Rohöl erhalten wird, das über Leitungen zu einer Raffinerie transportiert wird und während der nachfolgenden Raffination des leichten Rohöls zu herkömmlichem Benzin die produzierte Menge an Benzin um die im wasserhaltigen Kohlenwasserstoffgemisch enthaltenen Kohlenwasserstoffe erhöht.

2. Verfahren nach Anspruch 1, **dadurch gekennzeichnet, dass** aus dem Erd- und/oder Erdölbegleitgas das erste Synthesegas wie folgt hergestellt wird:
a1) das Begleitgas wird entschwefelt und mit Prozesskondensat und Dampf gesättigt;
a2) das gemäß Verfahrensschritt a1) erhaltene Prozessgas wird in ein Gasgemisch aus Methan, Kohlendioxid und Kohlenmonoxid vorgespalten;
a3) das vorgespaltene Gasgemisch wird unter erhöhter Temperatur und unter Druck in einem Steamreformer katalytisch in ein erstes Synthesegas umgewandelt, das abgekühlt und mittels eines Kompressors komprimiert wird.

3. Verfahren nach Anspruch 1, **dadurch gekennzeichnet, dass** aus der Teilmenge an schweren Rohöl das zweite Synthesegas wie folgt hergestellt wird:
b1) aufbereitetes Rohöl wird erhitzt und mit vorgewärmtem Sauerstoff, der in einer Luftzerlegungsanlage gewonnen wird, sowie Prozessdampf vermischt,
b2) dieses Gemisch wird in einem POX-Reaktor mittels nichtkatalytischer partieller Oxidation in CO und Wasserstoff gespalten,
b3) nachfolgendes Quenchen mit Wasser und Wärmeabfuhr über eine Abhitzestrecke und
b4) abschließendes Abtrennen von Schwefelwasserstoffverbindungen aus dem Prozessgas.

4. Verfahren nach einem der Ansprüche 1 bis 3, **dadurch gekennzeichnet, dass** die Umwandlung des Synthesegases in Methanol in einem dualen System, bestehend aus einem wassergekühlten und einem gasgekühlten Reaktor bei Temperaturen von 220 bis 260 °C vorgenommen wird.

5. Verfahren nach einem der Ansprüche 1 bis 4, **dadurch gekennzeichnet, dass** sich das erste und das zweite Synthesegas in ihrer Zusammensetzung unterscheiden.

6. Verfahren nach einem der Ansprüche 1 bis 5, **dadurch gekennzeichnet, dass** das Mischungsverhältnis zwischen dem ersten und zweiten Synthesegas auf das in der Methanolsynthese erforderliche Verhältnis von Wasserstoff zu Kohlenmonoxid abgestimmt wird, wobei die aus dem Begleitgas stammende Menge an Synthesegas den größeren Anteil bildet.

7. Verfahren nach einem der Ansprüche 1 bis 6, **dadurch gekennzeichnet, dass** während der Methanolsynthese eine Teilmenge an Synthesegas ausgekreist, im Kreislauf gefahren und dabei auf den erforderlichen Betriebsdruck komprimiert wird.

8. Verfahren nach einem der Ansprüche 1 bis 7, **dadurch gekennzeichnet, dass** das kondensierte Rohmethanol entgast und nachfolgend von niedrig siedenden und höher siedenden Verbindungen gereinigt wird, wobei die destillative Reinigung bei Temperaturen von 70 bis 140 °C in zwei Kolonnen vorgenommen wird und sich im Methanol ein Restwassergehalt von mindestens 4 % einstellt.

9. Verfahren nach einem der Ansprüche 1 bis 8, **dadurch gekennzeichnet, dass** das im Festbettreaktor anfallende Dimethylether/Methanol/Wasser-Gemisch mit Recyclegas zur Temperatureinstellung versetzt wird.

10. Verfahren nach einem der Ansprüche 1 bis 9, **dadurch gekennzeichnet, dass** das gebildete Kohlenwasserstoffgemisch unmittelbar, im Bereich der Erdöllagerstätte, entweder dem bereits geförderten und/oder dem noch unterirdisch gelagerten schweren Rohöl über das Bohrloch zugeführt wird.

11. Verfahren nach einem der Ansprüche 1 bis 10, **dadurch gekennzeichnet, dass** das gebildete Kohlenwasserstoffgemisch über ein Spülrohr in das Bohrloch eingebracht wird.

12. Verfahren nach einem der Ansprüche 1 bis 11, **dadurch gekennzeichnet, dass** das gebildete Kohlenwasserstoffgemisch vor dem Inkontaktbringen mit dem schweren Rohöl entwässert und entgast wird.

13. Verfahren nach einem der Ansprüche 1 bis 12, **dadurch gekennzeichnet, dass** aufbereitetes Kohlenwasserstoffgemisch, das entwässert und entgast ist, vor oder nach der zentralen Ölaufarbeitung dem schweren Rohöl zugesetzt wird.

14. Verfahren nach einem der Ansprüche 1 bis 13, **dadurch gekennzeichnet, dass** nicht aufbereitetes Kohlenwasserstoffgemisch vor der zentralen Ölaufarbeitung dem schweren Rohöl zugesetzt wird.

15. Verfahren nach einem der Ansprüche 1 bis 14, **dadurch gekennzeichnet, dass** nach der Abtrennung von Wasser und Ölbegleitgas aus dem Rohöl diesem eine weitere Menge an aufbereitetem Kohlenwasserstoffgemisch zugesetzt wird, die in Abhängigkeit von der Viskosität des Schweröls so dosiert wird, dass ein leichtes Rohöl gebildet wird.

16. Verfahren nach einem der Ansprüche 1 bis 15, **dadurch gekennzeichnet, dass** aufbereitetes Kohlenwasserstoffgemisch auf der Saugseite der zum Transport des Rohöls eingesetzten Pumpe zugeführt wird.

17. Verfahren nach einem der Ansprüche 1 bis 16, **dadurch gekennzeichnet, dass** die Viskosität des geförderten schweren Rohöls gemessen und in Abhängigkeit vom aktuellen Messergebnis die Menge an Kohlenwasserstoffgemisch dosiert zugesetzt wird, um leichtes Rohöl zu erhalten.

18. Verfahren nach einem der Ansprüche 1 bis 17, **dadurch gekennzeichnet, dass** aufbereitetes Kohlenwasserstoffgemisch und schweres Rohöl in einer separaten Mischeinrichtung vermischt werden.

## Claims

1. A method for improving the transportability of heavy crude oil, wherein a viscosity-reducing agent is added to the heavy crude oil, **characterized in that** the agent is a water-containing hydrocarbon mixture with a chain length of predominantly C4 to C12 that contains no oxygen-containing compounds and is prepared from generated natural gas and/or crude oil associated gas as well as from a sub-quantity of heavy crude oil, wherein a first synthesis gas and a second synthesis gas are produced in parallel, in each case as intermediate products, from natural gas and/or crude oil associated gas and from the branched-off sub-quantity of heavy crude oil, are merged, mixed in a mixer and subsequently compressed, and then the synthesis gas is catalytically converted into methanol, and this is converted by condensation into a methanol/water mixture (crude methanol), which is processed into a distillate with a high water and alcohol content of greater than 90% and is catalytically converted into a dimethyl ether/methanol/water mixture, and the water-containing hydrocarbon mixture with a chain length of C4 to C12 is formed by dehydration, which is added to the heavy crude oil either in an untreated form or subsequent to degassing and/or dewatering, thereby obtaining, from the heavy crude oil, a crude oil that is light in its quality and that is transported via pipelines to a refinery and that increases the produced quantity of gasoline by the hydrocarbons contained in the water-containing hydrocarbon mixture during the subsequent refining process of the light crude oil into conventional gasoline.

2. The method according to claim 1, **characterized in that** the first synthesis gas is produced from the natural and/or crude oil associated gas as follows:
a1) the associated gas is desulfurized and saturated with process condensate and steam;
a2) the process gas obtained in accordance with process step a1) is pre-cracked into a gas mixture of methane, carbon dioxide and carbon monoxide;
a3) the pre-cracked gas mixture is catalytically converted, under an elevated temperature and under pressure in a steam reformer, into a first synthesis gas that is cooled down and compressed by means of a compressor.

3. The method according to claim 1, **characterized in that** the second synthesis gas is produced from the sub-quantity of heavy crude oil as follows:
b1) treated crude oil is heated and mixed with pre-heated oxygen obtained in an air separation plant and process steam,
b2) this mixture is cracked in a POX reactor into CO and hydrogen by means of non-catalytic partial oxidation,
b3) subsequent quenching with water and heat dissipation via a waste heat section, and
b4) subsequent separation of hydrogen sulfide compounds from the process gas.

4. The method according to any one of the claims 1 to 3, **characterized in that** the conversion of the synthesis gas into methanol is carried out in a dual system consisting of a water-cooled and a gas-cooled reactor at temperatures of 220 to 260°C.

5. The method according to any one of the claims 1 to 4, **characterized in that** the first and the second synthesis gases differ with regard to their composition.

6. The method according to any one of the claims 1 to 5, **characterized in that** the mixing ratio between the first and the second synthesis gases is adapted to the ratio of hydrogen to carbon monoxide required in the methanol synthesis, wherein the quantity of synthesis gas coming from the associated gas forms the larger proportion.

7. The method according to any one of the claims 1 to 6, **characterized in that** during the methanol synthesis, a sub-quantity of synthesis gas is purged, circulated and, in the process, compressed to the required operating pressure.

8. The method according to any one of the claims 1 to 7, **characterized in that** the condensed crude methanol is degassed and then purified from low-boiling and higher-boiling compounds, wherein the purification by distillation is carried out at temperatures of 70 to 140°C in two columns and a residual water content of at least 4% in the methanol is reached.

9. The method according to any one of the claims 1 to 8, **characterized in that** recycle gas is added to the dimethyl ether/methanol/water mixture generated in the fixed-bed reactor for adjusting the temperature.

10. The method according to any one of the claims 1 to 9, **characterized in that** the formed hydrocarbon mixture is immediately, in the region of the oil deposit, supplied either to the heavy crude oil that has already been extracted and/or is still deposited underground via the borehole.

11. The method according to any one of the claims 1 to 10, **characterized in that** the formed hydrocarbon mixture is introduced into the borehole via a flushing pipe.

12. The method according to any one of the claims 1 to 11, **characterized in that** the formed hydrocarbon mixture is dewatered and degassed prior to being brought into contact with the heavy crude oil.

13. The method according to any one of the claims 1 to 12, **characterized in that** processed hydrocarbon mixture, which is dewatered and degassed, is added to the heavy crude oil before or after the central oil processing.

14. The method according to any one of the claims 1 to 13, **characterized in that** non-processed hydrocarbon mixture is added to the heavy crude oil before the central oil processing.

15. The method according to any one of the claims 1 to 14, **characterized in that** subsequent to the separation of water and oil associated gas from the crude oil, another quantity of processed hydrocarbon mixture is added thereto, which, depending on the viscosity of the heavy oil, is dosed in such a manner that a light crude oil is formed.

16. The method according to any one of the claims 1 to 15, **characterized in that** processed hydrocarbon mixture is supplied on the suction side of the pump used for transporting the crude oil.

17. The method according to any one of the claims 1 to 16, **characterized in that** the viscosity of the produced heavy crude oil is measured and the quantity of hydrocarbon mixture is added in a dosed manner, depending on the current measurement result, in order to obtain a light crude oil.

18. The method according to any one of the claims 1 to 17, **characterized in that** processed hydrocarbon mixture and heavy crude oil before are mixed in a separate mixing device.

## Revendications

1. Procédé destiné à améliorer la transportabilité de pétrole brut lourd, dans lequel un agent réducteur de viscosité est ajouté au pétrole brut lourd, **caractérisé par le fait que** ledit agent est un mélange d'hydrocarbures contenant de l'eau et ayant une longueur de chaîne principalement en C4 à C12 qui ne contient pas de composés contenant de l'oxygène, et est préparé, au niveau d'un gisement de pétrole, à partir de gaz naturel produit et/ou de gaz associé au pétrole ainsi qu'à partir d'une partie de pétrole brut lourd, dans lequel, parallèlement, on prépare, à partir de gaz naturel et/ou de gaz associé au pétrole ainsi que de la partie prélevée de pétrole brut lourd, respectivement en tant qu'intermédiaires, un premier gaz de synthèse et un deuxième gaz de synthèse qui sont réunis, mélangés dans un mélangeur et, puis, comprimés, et, ci-après, le gaz de synthèse est converti de façon catalytique en méthanol et celui-ci est converti par condensation en un mélange méthanol-eau (méthanol brut) qui est traité pour obtenir un distillat ayant une forte teneur en eau et en alcool supérieure à 90 % et est converti façon catalytique en un mélange de diméthyléther/méthanol/eau, et le mélange d'hydrocarbures contenant de l'eau et ayant une longueur de chaîne de C4 à C12 est formé par déshydratation, qui est ajouté au pétrole brut lourd soit sans être traité soit après le dégazage et/ou la déshydratation, ce par quoi on obtient à partir du pétrole brut lourd un pétrole brut léger en qualité qui est transporté par des conduites à une raffinerie, et, pendant l'affinage subséquent du pétrole brut léger destiné à obtenir de l'essence conventionnelle, la quantité produite d'essence est augmentée des hydrocarbures contenus dans le mélange d'hydrocarbures contenant de l'eau.

2. Procédé selon la revendication 1, **caractérisé par le fait que** le premier gaz de synthèse est produit comme suit à partir du gaz naturel et/ou du gaz associé au pétrole:
a1) le gaz associé est désulfuré et saturé avec du condensat de processus et de la vapeur,
a2) le gaz de processus obtenu selon l'étape a1) est pré-séparé en un mélange gazeux de méthane, de dioxyde de carbone et de monoxyde de carbone,
a3) le mélange gazeux pré-séparé est converti de façon catalytique, à température élevée et sous pression, dans un reformeur à la vapeur, en un premier gaz de synthèse qui est refroidi et comprimé en utilisant un compresseur.

3. Procédé selon la revendication 1, **caractérisé par le fait que** le deuxième gaz de synthèse est produit comme suit à partir de la partie de pétrole brut lourd:
b1) du pétrole brut traité est chauffé et est mélangé avec de l'oxygène préchauffé obtenu dans une installation de décomposition d'air ainsi qu'avec de la vapeur de processus,
b2) ce mélange est décomposé en CO et en hydrogène, dans un réacteur à l'oxydation partielle, au moyen d'une oxydation partielle non catalytique,
b3) un quench subséquent avec de l'eau et l'évacuation de la chaleur via un trajet à chaleur perdue, et
b4) la séparation finale de composés de sulfure d'hydrogène dudit gaz de processus.

4. Procédé selon l'une quelconque des revendications 1 à 3, **caractérisé par le fait que** la conversion du gaz de synthèse en méthanol est réalisée dans un système dual se composant d'un réacteur refroidi à l'eau et d'un réacteur refroidi au gaz, à des températures comprises entre 220 et 260 °C.

5. Procédé selon l'une quelconque des revendications 1 à 4, **caractérisé par le fait que** les premier et deuxième gaz de synthèse diffèrent par leur composition.

6. Procédé selon l'une quelconque des revendications 1 à 5, **caractérisé par le fait que** le rapport de mélange entre le premier et le deuxième gaz de synthèse est adapté au rapport de l'hydrogène au monoxyde de carbone, qui est requis dans la synthèse de méthanol, dans lequel la quantité de gaz de synthèse provenant du gaz associé constitue la proportion plus grande.

7. Procédé selon l'une quelconque des revendications 1 à 6, **caractérisé par le fait que**, durant la synthèse de méthanol, une partie de gaz de synthèse est retirée du circuit, est mise en circulation et est ainsi comprimée à la pression de fonctionnement requise.

8. Procédé selon l'une quelconque des revendications 1 à 7, **caractérisé par le fait que** le méthanol brut condensé est dégazé et ensuite purifié de composés à bas point d'ébullition et à point d'ébullition plus élevé, dans lequel la purification par voie de distillation est effectuée à des températures comprises entre 70 et 140 °C en deux colonnes, et une teneur en eau résiduelle d'au moins 4 % s'établit dans le méthanol.

9. Procédé selon l'une quelconque des revendications 1 à 8, **caractérisé par le fait que** le mélange de diméthyléther/méthanol/eau produit dans le réacteur à lit fixe est mélangé avec du gaz de recyclage pour le réglage de la température.

10. Procédé selon l'une quelconque des revendications 1 à 9, **caractérisé par le fait que** le mélange d'hydrocarbures formé est amené directement, au niveau du gisement de pétrole, par le trou de sondage, soit au pétrole brut lourd déjà produit et/ou au pétrole brut lourd stocké encore sous terre.

11. Procédé selon l'une quelconque des revendications 1 à 10, **caractérisé par le fait que** le mélange d'hydrocarbures formé est introduit par l'intermédiaire d'un tuyau de rinçage dans le trou de sondage.

12. Procédé selon l'une quelconque des revendications 1 à 11, **caractérisé par le fait que** le mélange d'hydrocarbures formé est déshydraté et dégazé avant la mise en contact avec le pétrole brut lourd.

13. Procédé selon l'une quelconque des revendications 1 à 12, **caractérisé par le fait que** du mélange d'hydrocarbures traité qui est déshydraté et dégazé est ajouté au pétrole brut lourd avant ou après le traitement central d'huile.

14. Procédé selon l'une quelconque des revendications 1 à 13, **caractérisé par le fait que** du mélange d'hydrocarbures non traité est ajouté au pétrole brut lourd avant le traitement central d'huile.

15. Procédé selon l'une quelconque des revendications 1 à 14, **caractérisé par le fait que**, après avoir séparé l'eau et le gaz associé à l'huile du pétrole brut, on ajoute à ce dernier une autre quantité de mélange d'hydrocarbures traité qui est dosée en fonction de la viscosité de l'huile lourde de telle manière qu'un pétrole brut léger est formé.

16. Procédé selon l'une quelconque des revendications 1 à 15, **caractérisé par le fait que** du mélange d'hydrocarbures traité est amené sur le côté d'aspiration à la pompe utilisée pour le transport du pétrole brut.

17. Procédé selon l'une quelconque des revendications 1 à 16, **caractérisé par le fait que** la viscosité du pétrole brut lourd produit est mesurée et que la quantité de mélange d'hydrocarbures est ajouté de manière dosée en fonction du résultat actuel de mesure afin d'obtenir du pétrole brut léger.

18. Procédé selon l'une quelconque des revendications 1 à 17, **caractérisé par le fait que** du mélange d'hydrocarbures traité et du pétrole brut lourd sont mélangés dans un dispositif mélangeur séparé.
